# EUROPEAN PATENT SPECIFICATION

(11) **EP 0 971 714 B1**
(45) Date of publication and mention of the grant of the patent: **09.10.2002**
(21) Application number: 98912108.2
(22) Date of filing: 30.03.1998
(51) Int. Cl.: A61K 31/635, A61K 31/415, A61P 25/28

(54) **METHOD OF USING CYCLOOXYGENASE-2 INHIBITORS IN THE TREATMENT AND PREVENTION OF DEMENTIA**
VERFAHREN ZUR VERWENDUNG VON CYCLOOXYGENASE-2 HEMMERN ZUR BEHANDLUNG UND VORBEUGUNG DER DEMENZ
PROCEDE SE RAPPORTANT A L'UTILISATION D'INHIBITEURS DE LA CYCLO-OXYGENASE-2 POUR LE TRAITEMENT ET LA PREVENTION DE LA DEMENCE

(30) Priority: 03.04.1997 US 43916 P
(43) Date of publication of application: 19.01.2000
(73) Proprietor: G.D. SEARLE & CO., Chicago, IL 60680-5110 (US)
(72) Inventor: NEEDLEMAN, Philip, Creve Coeur, MO 63141 (US)
(74) Representative: Ebner von Eschenbach, Jennifer
(86) International application number: US9806143
(87) International publication number: WO98043648

(56) References cited:
- WO-A-95/15316
- WO-A-96/03385
- WO-A-96/09293
- WO-A-96/09304
- WO-A-96/11676
- WO-A-96/38418
- WO-A-96/38442
- WO-A-96/41625
- WO-A-96/41626
- WO-A-96/41645
- WO-A-97/38986
- J.R. VANE ET AL.: "CYCLOOXYGENASES 1 AND 2" ANNU. REV. PHARMACOL. TOXICOL., vol. 38, 1998, pages 97-120, XP002074177
- J.Y. JOUZEAU ET AL.: "CYCLO-OXYGENASE ISOENZYMES" DRUGS, vol. 53, no. 4, April 1997, pages 563-582, XP002074178
- J. VAN RYN ET AL.: "SELECTIVE CYCLOOXYGENASE-2 INHIBITORS: PHARMACOLOGY, CLINICAL EFFECTS AND THERAPEUTIC POTENTIAL" EXP. OPIN. INVEST. DRUGS, vol. 6, no. 5, 1997 - March 1997, pages 609-614, XP002074179

## Description

### Field of the Invention

This invention is in the field of the prevention and treatment of dementia. More specifically, this invention relates to the use of cyclooxygenase-2 inhibitors or salts thereof for preparating a medicament for preventing and treating dementia.

### Background of the Invention

Prostaglandins play a major role in the inflammation process and the inhibition of prostaglandin production, especially production of PGG₂, PGH₂ and PGE₂, has been a common target of anti-inflammatory drug discovery. However, common non-steroidal anti-inflammatory drugs (NSAID's) that are active in reducing the prostaglandin-induced pain and swelling associated with the inflammation process are also active in affecting other prostaglandin-regulated processes not associated with the inflammation process. Thus, use of high doses of most common NSAID's can produce severe side effects, including life threatening ulcers, that limit their therapeutic potential. An alternative to NSAID's is the use of corticosteroids, which also produce adverse effects, especially when long term therapy is involved.

NSAIDs have been found to prevent the production of prostaglandins by inhibiting enzymes in the human arachidonic acid/prostaglandin pathway, including the enzyme cyclooxygenase (COX). The recent discovery of an inducible enzyme associated with inflammation (named "cyclooxygenase-2 (COX-2)" or "prostaglandin G/H synthase II") provides a viable target of inhibition which more effectively reduces inflammation and produces fewer and less drastic side effects.

Dementia, characterized by memory loss, confusion, and disorientation is suffered by fifteen per cent of the American elderly population. Of that number, about sixty per cent suffer from the progressive mental deterioration known as Alzheimer's disease.

Alzheimer's disease causes the death of brain cells, often begins in the portion of the brain responsible for memory control. The onset is normally gradual, often symptomized by the loss of short term memory. In the late stages of the disease the patient becomes disoriented and ultimately becomes unable to care for themselves.

United States patent No. 5,192,753 (McGeer et al.) describes the use of the NSAID indomethacin for treatment of dementia. WO patent publication WO94/13635 (published June 23, 1994) describes the use of specific COX-2 compounds for the treatment of Alzheimer's disease.

[Pyrazol-1-yl]benzenesulfonamides have been described in WO956/15316 as inhibitors of cyclooxygenase-2 and have shown promise in the treatment of inflammation, arthritis, and pain, with minimal side effects in pre-clinical and clinical trials. However, their use for treating central nervous system disorders, including dementia or specifically for treating or preventing other Alzheimer's disease has not been previously described.

WO 96 38418 A, to Searle & Co., published December 5, 1996, concerns the field of anti-inflammatory pharmaceutical agents and specifically relates to compounds, compositions and methods for treating disorders mediated by cyclooxygenase-2 or 5-lipoxygenase such as inflammation.

J.Y. Joyzeau et al., Cyclooxygenase-2 isoenzymes: Drugs 53(4): 563-582 (1997). This article discusses the discovery of COX isoenzymes and the effect of selective COPX-1 and COX-2 inhibitors on the field of NSAID pharmaceuticals.

J. Van Ryn et al., Selective cyclooxygenase-2 inhibitors: pharmacology clinical effects and therapeutic potential. Exp. Opin. Invest. Drugs 6(5):609-614 (1997). This article discusses a wide variety of topics relating to COX-2 inhibitors, including their discovery, relevance to inflammation research, structure, selectivity, potential new indications, and a variety of side effects.

WO 96 38442 A, to Searle & Co., published December 5, 1996. This invention is in the field of anti-inflammatory pharmaceutical agents and specifically relates to compounds, compositions and methods for treating disorders mediated by COX-2 or 5-lipoxygenase, such as inflammation.

### Detailed Description of the Invention

The present invention provides the use of a therapeutically effective amount of a cyclooxygenase-2 inhibitor of Formule I or salt thereof for the preparation of a medicament for treating or preventing dementia.

The term "treatment" includes partial or total inhibition of the dementia, including Alzheimer's disease, vascular dementia, multi-infarct dementia, pre-senile dementia, alcoholic dementia, and senile dementia.

The term "prevention" includes either preventing the onset of clinically evident dementia altogether or preventing the onset of a preclinically evident stage of dementia in individuals at risk. Also intended to be encompassed by this definition is the prevention of initiation brain cell death or to arrest or reverse the progression of Alzheimer's disease symptoms. This includes prophylactic treatment of those at risk of developing dementia.

The phrase "therapeutically-effective" is intended to qualify the amount of each agent which will achieve the goal of improvement in disease severity and the frequency of incidence over treatment of each agent by itself, while avoiding adverse side effects typically associated with alternative therapies.

The term "subject" for purposes of treatment includes any human or animal subject who has any one of the known dementia, and preferably is a human subject. For methods of prevention, the subject is any human or animal subject, and preferably is a human subject who is at risk for dementia. The subject may be at risk due to exposure to head injury, being exposed to other enviromnmental factors associated with Alzheimer's disease and being genetically predisposed to have the dementia.

In the method above, dementia includes Alzheimer's disease, vascular dementia, multi-infarct dementia, pre-senile dementia, alcoholic dementia, and senile dementia.

Inhibitors of the cyclooxygenase pathway in the metabolism of arachidonic acid used in the prevention and treatment of dementia may inhibit enzyme activity through a variety of mechanisms. By the way of example, the inhibitors used in the methods described herein may block the enzyme activity directly by acting as a substrate for the enzyme. The use of cyclooxygenasse-2 selective inhibitors is highly advantageous in that they minimize the gastric side effects that can occur with non-selective NSAID's, especially where prolonged prophylactic treatment is expected.

The term "cyclooxygenase-2 inhibitor" denotes a compound able to inhibit cyclooxygenase-2 without significant inhibition of cyclooxygenase-1.

The cyclooxygenase-2 inhibitor is selected from compounds of Formula I 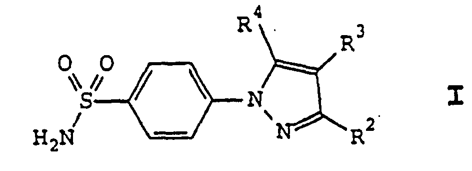 wherein R² is selected from hydrido, alkyl, haloalkyl, alkoxycarbonyl, cyano, cyanoalkyl, carboxyl, aminocarbonyl, alkylaminocarbonyl, cycloalkylaminocarbonyl, arylaminocarbonyl, carboxyalkylaminocarbonyl, carboxyalkyl, aralkoxycarbonylalkylaminocarbonyl, alkoxycarbonylcyanoalkenyl and hydroxyalkyl;
wherein R³ is selected from hydrido, alkyl, cyano, hydroxyalkyl, cycloalkyl, alkylsulfonyl and halo; and
wherein R⁴ is selected from aralkehyl, aryl, cycloalkyl, cycloalkenyl and heterocyclic; wherein R⁴ is optionally substituted at a substitutable position with one or more radicals selected from halo, alkylthio, alkylsulfonyl, cyano, nitro, haloalkyl, alkyl, hydroxyl, alkenyl, hydroxyalkyl, carboxyl, cycloalkyl, alkylamino, dialkylamino, alkoxycarbonyl, aminocarbonyl, alkoxy, haloalkoxy, sulfamyl, heterocyclic and amino;
or a pharmaceutically-acceptable salt or derivative thereof.

A class of compounds of particular interest consists of those compounds of Formula I wherein R² is selected from hydrido, lower alkyl, lower haloalkyl, lower alkoxycarbonyl, cyano, lower cyanoalkyl, carboxyl, aminocarbonyl, lower alkylaminocarbonyl, lower cycloalkylaminocarbonyl, arylaminocarbonyl, lower carboxyalkylaminocarbonyl, lower aralkoxycarbonylalkylaminocarbonyl, lower carboxyalkyl, lower alkoxycarbonylcyanoalkenyl and lower hydroxyalkyl; wherein R³ is selected from hydrido, lower alkyl, cyano, lower hydroxyalkyl, lower cycloalkyl, lower alkylsulfonyl and halo; and wherein R⁴ is selected from aralkenyl, aryl, cycloalkyl, cycloalkenyl and heterocyclic; wherein R⁴ is optionally substituted at a substitutable position with one or more radicals selected from halo, lower alkylthio, lower alkylsulfonyl, cyano, nitro, lower haloalkyl, lower alkyl, hydroxyl, lower alkenyl, lower hydroxyalkyl, carboxyl, lower cycloalkyl, lower alkylamino, lower dialkylamino, lower alkoxycarbonyl, aminocarbonyl, lower alkoxy, lower haloalkoxy, sulfamyl, five or six membered heterocyclic and amino; or a pharmaceutically-acceptable salt or derivative thereof.

A family of specific compounds of particular interest within Formula I consists of compounds, pharmaceutically-acceptable salts and derivatives thereof as follows:
4-[5-(4-chlorophenyl)-3-(trifluoromethyl)-1H-pyrazol-1-yl]benzenesulfonamide;
4-[5-phenyl-3-(trifluoromethyl)-1H-pyrazol-1-yl]benzenesulfonamide;
4-[5-(4-fluorophenyl)-3-(trifluoromethyl)-1H-pyrazol-1-yl]benzenesulfonamide;
4-[5-(4-methoxyphenyl)-3-(trifluoromethyl)-1H-pyrazol-1-yl]benzenesulfonamide;
4-[5-(4-chlorophenyl)-3-(difluoromethyl)-1H-pyrazol-1-yl]benzenesulfonamide;
4-[5-(4-methylphenyl)-3-(trifluoromethyl)-1H-pyrazol-1-yl]benzenesulfonamide;
4-[4-chloro-5-(4-chlorophenyl)-3-(trifluoromethyl)-1H-pyrazol-1-yl]benzenesulfonamide;
4-[3-(difluoromethyl)-5-(4-methylphenyl)-1H-pyrazol-1-yl]benzenesulfonamide;
4-[3-(difluoromethyl)-5-phenyl-1H-pyrazol-1-yl]benzenesulfonamide;
4-[3-(difluoromethyl)-5-(4-methoxyphenyl)-1H-pyrazol-1-yl]benzenesulfonamide;
4-[3-cyano-5-(4-fluorophenyl)-1H-pyrazol-1-yl]benzenesulfonamide;
4-[3-(difluoromethyl)-5-(3-fluoro-4-methoxyphenyl)-1H-pyrazol-1-yl]benzenesulfonamide;
4-[5-(3-fluoro-4-methoxyphenyl)-3-(trifluoromethyl)-1H-pyrazol-1-yl]benzenesulfonamide;
4-[4-chloro-5-phenyl-1H-pyrazol-1-yl]benzenesulfonamide;
4-[5-(4-chlorophenyl)-3-(hydroxymethyl)-1H-pyrazol-1-yl]benzenesulfonamide; and
4-[5-(4-(N,N-dimethylamino)phenyl)-3-(trifluoromethyl)-1H-pyrazol-1-yl]benzenesulfonamide.

A family of specific compounds of more particular interest within Formula I consists of compounds and pharmaceutically-acceptable salts or derivatives thereof as follows:
4-[5-(4-methylphenyl)-3-(trifluoromethyl)-1H-pyrazol-1-yl]benzenesulfonamide;
4-[5-(4-chlorophenyl)-3-(difluoromethyl)-1H-pyrazol-1-yl]benzenesulfonamide; and
4-[5-(3-fluoro-4-methoxyphenyl)-3-(difluoromethyl)-1H-pyrazol-1-yl]benzenesulfonamide.

The compounds utilized in the methods of the present invention may be present in the form of free bases or pharmaceutically acceptable acid addition salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, stearic, algenic, β-hydroxybutyric, salicylic, galactaric and galacturonic acid. Suitable pharmaceutically-acceptable base addition salts of compounds of Formula I include metallic salts made from aluminum, calcium, lithium, magnesium, potassium, sodium and zinc or organic salts made from N,N'-dibenzylethylenediamine, chloroprocaine, choline, diethanolamine, ethylenediamine, meglumine (N-methylglucamine) and procaine. All of these salts may be prepared by conventional means from the corresponding compound of Formula I by reacting, for example, the appropriate acid or base with the compound of Formula I.

### Materials and Methods

A transgenic mouse model for Alzheimer;s has been described by Hsiao et al. (Science, (1996)]. The cyclooxygenasse-2 inhibitors should be active, at a dose of 20 mg/kg.

The active compounds of the present invention may be administered by any suitable route known to those skilled in the art, preferably in the form of a pharmaceutical composition adapted to such a route, and in a dose effective for the treatment intended. The active compounds and composition may, for example, be administered orally, intravascularly, intraperitoneally, intranasal, intrabronchial, subcutaneously, intramuscularly or topically (including aerosol).

The administration of the present invention may be for either prevention or treatment purposes. The methods and compositions used herein may be used alone or in conjunction with additional therapies known to those skilled in the art in the prevention or treatment of dementia. Alternatively, the methods and compositions described herein may be used as adjunct therapy. By way of example, the cyclooxygenase-2 inhibitor may be administered alone or in conjunction with other antineoplastic agents or other growth inhibiting agents or other drugs or nutrients.

The phrase "adjunct therapy" (or "combination therapy"), in defining use of a cyclooxygenase-2 inhibitor agent and another pharmaceutical agent, is intended to embrace administration of each agent in a sequential manner in a regimen that will provide beneficial effects of the drug combination, and is intended as well to embrace co-administration of these agents in a substantially simultaneous manner, such as in a single formulation having a fixed ratio of these active agents, or in multiple, separate formulations for each agent. The present invention also comprises a pharmaceutical composition for the adjunct prevention and treatment of dementia, comprising a therapeutically-effective amount of a compound of Formula I in association with at least one pharmaceutically-acceptable carrier, adjuvant or diluent (collectively referred to herein as "carrier" materials) and, other antidementia agents or other growth inhibiting agents or other drugs or nutrients.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable carrier.

For intravenous, intramuscular, subcutaneous, or intraperitoneal administration, the compound may be combined with a sterile aqueous solution which is preferably isotonic with the blood of the recipient. Such formulations may be prepared by dissolving solid active ingredient in water containing physiologically compatible substances such as sodium chloride, glycine, and the like, and having a buffered pH compatible with physiological conditions to produce an aqueous solution, and rendering said solution sterile. The formulations may be present in unit or multi-dose containers such as sealed ampoules or vials.

Formulations suitable for parenteral administration conveniently comprise a sterile aqueous preparation of the active compound which is preferably made isotonic. Preparations for injections may also be formulated by suspending or emulsifying the compounds in non-aqueous solvent, such as vegetable oil, synthetic aliphatic acid glycerides, esters of higher aliphatic acids or propylene glycol.

Formulations for topical use include known gels, creams, oils, and the like. For aerosol delivery, the compounds may be formulated with known aerosol exipients, such as saline, and administered using commercially available nebulizers. Formulation in a fatty acid source may be used to enhance biocompatibility.

For rectal administration, the active ingredient may be formulated into suppositories using bases which are solid at room temperature and melt or dissolve at body temperature. Commonly used bases include cocoa butter, glycerinated gelatin, hydrogenated vegetable oil, polyethylene glycols of various molecular weights, and fatty esters of polyethylene stearate.

The dosage form and amount can be readily established by reference to known central nervous system treatment or prophylactic regiments. The amount of therapeutically active compound that is administered and the dosage regimen for treating a disease condition with the compounds and/or compositions of this invention depends on a variety of factors, including the age, weight, sex and medical condition of the subject, the severity of the disease, the route and frequency of administration, and the particular compound employed, as well as the pharmacokinetic properties of the individual treated, and thus may vary widely. The dosage will generally be lower if the compounds are administered locally rather than systemically, and for prevention rather than for treatment. Such treatments may be administered as often as necessary and for the period of time judged necessary by the creating physician. One of skill in the art will appreciate that the dosage regime or therapeutically effective amount of the inhibitor to be administrated may need to be optimized for each individual. The pharmaceutical compositions may contain active ingredient in the range of about 0.1 to 2000 mg, preferably in the range of about 0.5 to 500 mg and most preferably between about 1 and 200 mg. A daily dose of about 0.01 to 100 mg/kg body weight, preferably between about 0.1 and about 50 mg/kg body weight and most preferably from about 1 to 20 mg/kg body weight, may be appropriate. The daily dose can be administered in one to four doses per day.

## Claims

1. The use of a compound of Formula I. 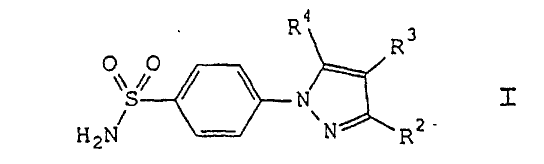 wherein R² is selected from hydrido, alkyl, haloalkyl, alkoxycarbonyl, cyano, cyanoalkyl, carboxyl, aminocarbonyl, alkylaminocarbonyl, cycloalkylaminocarbonyl, arylaminocarbonyl, carboxyalkylaminocarbonyl, carboxyalkyl, aralkoxycarbonylalkylaminocarbonyl, alkoxycarbonylcyanoalkenyl and hydroxyalkyl;
wherein R³ is selected from hydrido, alkyl, cyano, hydroxyalkyl, cycloalkyl, alkylsulfonyl and halo; and
wherein R⁴ is selected from aralkenyl, aryl, cycloalkyl, cycloalkenyl and heterocyclic; wherein R⁴ is optionally substituted at a substitutable position with one or more radicals selected from halo, alkylthio, alkylsulfonyl, cyano, nitro, haloalkyl, alkyl, hydroxyl, alkenyl, hydroxyalkyl, carboxyl, cycloalkyl, alkylamino, dialkylamino, alkoxycarbonyl, aminocarbonyl, alkoxy, haloalkoxy, sulfamyl, heterocyclic and amino;
or a pharmaceutically-acceptable salt thereof, for preparing a medicament for treating an dementia in a subject.

2. The use according Claim 1 wherein R² is selected from hydrido, C₁-C₁₀-alkyl, C₁-C₆-haloalkyl, C₁-C₆-alkoxycarbonyl, cyano, C₁-C₆-cyanoalkyl, carboxyl, aminocarbonyl, N-C₁-C₆-alkylaminocarbonyl, C₃-C₇-cycloalkylaminocarbonyl, arylaminocarbonyl, carboxy-C₁-C₆-alkylaminocarbonyl, aryl-C₁-C₆-alkoxycarbonylalkylaminocarbonyl, carboxy-C₁-C₆-alkyl, C₁-C₆-alkoxycarbonylcyanoalkenyl and C₁-C₆-hydroxyalkyl;
wherein R³ is selected from hydrido, C₁-C₁₀-alkyl, cyano, C₁-C₆-hydroxyalkyl, C₃-C₇-cycloalkyl, C₁-C₆-alkylsulfonyl and halo; and wherein R⁴ is selected from aralkenyl, aryl, cycloalkyl, cycloalkenyl and heterocyclic; wherein R⁴ is optionally substituted at a substitutable position with one or more radicals selected from halo, C₁-C₆-alkylthio, C₁-C₆-alkylsulfonyl, cyano, nitro, C₁-C₆-haloalkyl, C₁-C₁₀-alkyl, hydroxyl, C₂-C₆-alkenyl, C₁-C₆-hydroxyalkyl, carboxyl, C₃-C₇-cycloalkyl, N-C₁-C₆-alkylamino, di-N-C₁-C₆-alkylamino, C₁-C₆-alkoxycarbonyl, aminocarbonyl, C₁-C₆-alkoxy, C₁-C₆-haloalkoxy, sulfamyl, five or six membered heterocyclic and amino; or a pharmaceutically-acceptable salt thereof.

3. The use according to Claim 2 wherein the compound is selected from compounds, and their pharmaceutically acceptable salts, of the group consisting of
4-[5-(4-chlorophenyl)-3-(trifluoromethyl)-1H-pyrazol-1-yl]benzenesulfonamide;
4-[5-phenyl-3-(trifluoromethyl)-1H-pyrazol-1-yl]benzenesulfonamide;
4-[5-(4-fluorophenyl)-3-(trifluoromethyl)-1H-pyrazol-1-yl]benzenesulfonamide;
4-[5-(4-methoxyphenyl)-3-(trifluoromethyl)-1H-pyrazol-1-yl]benzenesulfonamide;
4-[5-(4-chlorophenyl)-3-(difluoromethyl)-1H-pyrazol-1-yl]benzenesulfonamide;
4- [5-(4-methylphenyl)-3-(trifluoromethyl)-1H-pyrazol-1-yl]benzenesulfonamide;
4-[4-chloro-5-(4-chlorophenyl)-3-(trifluoromethyl)-1H-pyrazol-l-yl]benzenesulfonamide;
4-[3-(difluoromethyl)-5-(4-methylphenyl)-1H-pyrazol-1-yl]benzenesulfonamide;
4-[3-(difluoromethyl)-5-phenyl-1H-pyrazol-1-yl]benzenesulfonamide;
4-[3-(difluoromethyl)-5-(4-methoxyphenyl)-1H-pyrazol-1-yl]benzenesulfonamide;
4-[3-cyano-5-(4-fluorophenyl)-1H-pyrazol-1-yl]benzenesulfonamide;
4-[3-(difluoromethyl)-5-(3-fluoro-4-methoxyphenyl)-1H-pyrazol-1-yl]benzenesulfonamide;
4-[5-(3-fluoro-4-methoxyphenyl)-3-(trifluoromethyl)-1H-pyrazol-1-yl]benzenesulfonamide;
4-[4-chloro-5-phenyl-1H-pyrazol-1-yl]benzenesulfonamide;
4-[5-(4-chlorophenyl)-3-(hydroxymethyl)-1H-pyrazol-1-yl]benzenesulfonamide; and
4-[5-(4-(N,N-dimethylamino)phenyl)-3-(trifluoromethyl)-1H-pyrazol-1-yl]benzenesulfonamide.

4. The use according to Claim 2 wherein the compound is 4-[5-(4-methylphenyl)-3-(trifluoromethyl)-1H-pyrazol-1-yl]benzenesulfonamide, or a pharmaceutically-acceptable salt thereof.

5. The use according to Claim 2 wherein the compound is 4-[5-(4-chlorophenyl)-3-(difluoromethyl)-1H-pyrazol-1-yl]benzenesulfonamide, or a pharmaceutically-acceptable salt thereof.

6. The use according to Claim 2 where the compound is 4-[5-(3-fluoro-4-methoxyphenyl)-3-(difluoromethyl)-1H-pyrazol-1-yl]benzenesulfonamide, or a pharmaceutically-acceptable salt thereof.

7. The use according to Claim 1 wherein the dementia is selected from Alzheimer's disease, vascular dementia, multi-infarct dementia, pre-senile dementia, alcoholic dementia, and senile dementia.

8. The use according to Claim 7 wherein the dementia is Alzheimer's disease.

9. The use of a compound of Formula I 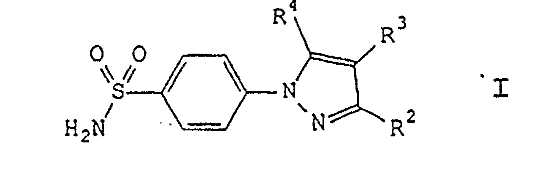 wherein R² is selected from hydrido, alkyl, haloalkyl, alkoxycarbonyl, cyano, cyanoalkyl, carboxyl, aminocarbonyl, alkylaminocarbonyl, cycloalkylaminocarbonyl, arylaminocarbonyl, carboxyalkylaminocarbonyl, carboxyalkyl, aralkoxycarbonylalkylaminocarbonyl, aminocarbonylalkyl, alkoxycarbonylcyanoalkenyl and hydroxyalkyl;
wherein R³ is selected from hydrido, alkyl, cyano, hydroxyalkyl, cycloalkyl, alkylsulfonyl and halo; and
wherein R⁴ is selected from aralkenyl, aryl, cycloalkyl, cycloalkenyl and heterocyclic; wherein R⁴ is optionally substituted at a substitutable position with one or more radicals selected from halo, alkylthio, alkylsulfonyl, cyano, nitro, haloalkyl, alkyl, hydroxyl, alkenyl, hydroxyalkyl, carboxyl, cycloalkyl, alkylamino, dialkylamino, alkoxycarbonyl, aminocarbonyl, alkoxy, haloalkoxy, sulfamyl, heterocyclic and amino;
or a pharmaceutically-acceptable salt thereof, for preparing a medicament for preventing a dementia selected from Alzheimer's disease, vascular dementia, multi-infarct dementia, pre-senile dementia, alcoholic dementia, and senile dementia.

10. The use according to Claim 9 wherein R² is selected from hydrido, C₁-C₁₀-alkyl, C₁-C₆-haloalkyl, C₁-C₆-alkoxycarbonyl, cyano, C₁-C₆-cyanoalkyl, carboxyl, aminocarbonyl, C₁-C₆-N-alkylaminocarbonyl, C₃-C₇-cycloalkylaminocarbonyl, arylaminocarbonyl, carboxy-C₁-C₆-alkylaminocarbonyl, aminocarbonyl-C₁-C₆-alkyl, aryl-C₁-C₆-alkoxycarbonylalkylaminocarbonyl, carboxy-C₁-C₆-alkyl, C₁-C₆-alkoxycarbonylcyanoalkenyl and C₁-C₆-hydroxyalkyl; wherein R³ is selected from hydrido, C₁-C₁₀-alkyl, cyano, C₁-C₆-hydroxyalkyl, C₃-C₇-cycloalkyl, C₁-C₆-alkylsulfonyl and halo; and wherein R⁴ is selected from aralkenyl, aryl, cycloalkyl, cycloalkenyl and heterocyclic; wherein R⁴ is optionally substituted at a substitutable position with one or more radicals selected from halo, C₁-C₆-alkylthio, C₁-C₆-alkylsulfonyl, cyano, nitro, C₁-C₆-haloalkyl, C₁-C₁₀-alkyl, hydroxyl, C₂-C₆-alkenyl, C₁-C₆-hydroxyalkyl, carboxyl, C₃-C₇-cycloalkyl, C₁-C₆-N-alkylamino, C₁-C₆-N-dialkylamino, C₁-C₆-alkoxycarbonyl, aminocarbonyl, C₁-C₆-alkoxy, C₁-C₆-haloalkoxy, sulfamyl, five or six membered heterocyclic and amino; or a pharmaceutically-acceptable salt thereof.

11. The use according to Claim 10 wherein the compound is selected from compounds, and their pharmaceutically acceptable salts, of the group consisting of
4-[5-(4-chlorophenyl)-3-(trifluoromethyl)-1H-pyrazol-1-yl]benzenesulfonamide;
4-[5-phenyl-3-(trifluoromethyl)-1H-pyrazol-1-yl]benzenesulfonamide;
4-[5-(4-fluorophenyl)-3-(trifluoromethyl)-1H-pyrazol-1-yl]benzenesulfonamide;
4-[5-(4-methoxyphenyl)-3-(trifluoromethyl)-1H-pyrazol-1-yl]benzenesulfonamide;
4-[5-(4-chlorophenyl)-3-(difluoromethyl)-1H-pyrazol-1-yl]benzenesulfonamide;
4-[5-(4-methylphenyl)-3-(trifluoromethyl)-1H-pyrazol-1-yl]benzenesulfonamide;
4-[4-chloro-5-(4-chlorophenyl)-3-(trifluoromethyl)-1H-pyrazol-1-yl]benzenesulfonamide;
4-[3-(difluoromethyl)-5-(4-methylphenyl)-1H-pyrazol-1-yl]benzenesulfonamide;
4-[3-(difluoromethyl)-5-phenyl-1H-pyrazol-1-yl]benzenesulfonamide;
4-[3-(difluoromethyl)-5-(4-methoxyphenyl)-1H-pyrazol-1-yl]benzenesulfonamide;
4-[3-cyano-5-(4-fluorophenyl)-1H-pyrazol-1-yl]benzenesulfonamide;
4-[3-(difluoromethyl)-5-(3-fluoro-4-methoxyphenyl)-1H-pyrazol-1-yl]benzenesulfonamide;
4-[5-(3-fluoro-4-methoxyphenyl)-3-(trifluoromethyl)-1H-pyrazol-1-yl]benzenesulfonamide;
4-[4-chloro-5-phenyl-1H-pyrazol-1-yl]benzenesulfonamide;
4-[5-(4-chlorophenyl)-3-(hydroxymethyl)-1H-pyrazol-1-yl]benzenesulfonamide; and
4-[5-(4-(N,N-dimethylamino)phenyl)-3-(trifluoromethyl)-1H-pyrazol-1-yl]benzenesulfonamide.

12. The useaccording to Claim 10 wherein the compound is 4-[5-(4-methylphenyl)-3-(trifluoromethyl)-1H-pyrazol-1-yl]benzenesulfonamide, or a pharmaceutically-acceptable salt thereof.

13. The use according to Claim 10 wherein the compound is 4-[5-(4-chlorophenyl)-3-(difluoromethyl)-1H-pyrazol-1-yl]benzenesulfonamide, or a pharmaceutically-acceptable salt thereof.

14. The use according to Claim 10 where the compound is 4-[5-(3-fluoro-4-methoxyphenyl)-3-(difluoromethyl)-1H-pyrazol-1-yl]benzenesulfonamide, or a pharmaceutically-acceptable salt thereof.

## Patentansprüche

1. Verwendung einer Verbindung der Formel I 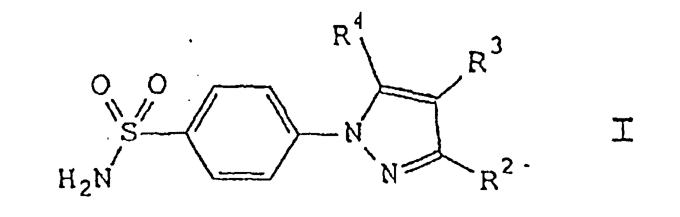 worin R² ausgewählt wird aus: Hydrido, Alkyl, Halogenalkyl, Alkoxycarbonyl, Cyano, Cyanoalkyl, Carboxyl, Aminocarbonyl, Alkylaminocarbonyl, Cycloalkylaminocarbonyl, Arylaminocarbonyl, Carboxyalkylaminocarbonyl, Carboxyalkyl, Aralkoxycarbonylalkylaminocarbonyl, Alkoxycarbonylcyanoalkenyl und Hydroxyalkyl;
worin R³ ausgewählt wird aus: Hydrido, Alkyl, Cyano, Hydroxyalkyl, Cycloalkyl, Alkylsulfonyl und Halogen; sowie
worin R⁴ ausgewählt wird aus: Aralkenyl, Aryl, Cycloalkyl, Cycloalkenyl und Heterocyclen; worin R⁴ wahlweise in einer substituierbaren Stelle substituiert ist mit einem oder mehreren Resten, ausgewählt aus Halogen, Alkylthio, Alkylsulfonyl, Cyano, Nitro, Halogenalkyl, Alkyl, Hydroxyl, Alkenyl, Hydroxyalkyl, Carboxyl, Cycloalkyl, Alkylamino, Dialkylamino, Alkoxycarbonyl, Aminocarbonyl, Alkoxy, Halogenalkoxy, Sulfamyl, Heterocyclen und Amino;
oder eines pharmazeutisch zulässigen Salzes davon zum Herstellen eines Medikamentes für die Behandlung einer Demenz in einem Patienten.

2. Verwendung nach Anspruch 1, bei welcher R² ausgewählt wird aus Hydrido, C₁-C₁₀-Alkyl, C₁-C₆-Halogenalkyl, C₁-C₆-Alkoxycarbonyl, Cyano, C₁-C₆-Cyanoalkyl, Carboxyl, Aminocarbonyl, N-C₁-C₆-Alkylaminocarbonyl, C₃-C₇-Cycloalkylaminocarbonyl, Arylaminocarbonyl, Carboxy-C₁-C₆-Alkylaminocarbonyl, Aryl-C₁-C₆-Alkoxycarbonylalkylaminocarbonyl, Carboxy-C₁-C₆-Alkyl, C₁-C₆-Alkoxycarbonylcyanoalkenyl und C₁-C₆-Hydroxyalkyl; worin R³ ausgewählt wird aus Hydrido, C₁-C₁₀-Alkyl, Cyano, C₁-C₆-Hydroxyalkyl, C₃-C₇-Cycloalkyl, C₁-C₆-Alkylsulfonyl und Halogen; und worin R⁴ ausgewählt wird aus Aralkenyl, Aryl, Cycloalkyl, Cycloalkenyl und Heterocyclen; worin R⁴ wahlweise an einer substituierbaren Stelle substituiert ist mit einem oder mehreren Resten, ausgewählt aus Halogen, C₁-C₆-Alkylthio, C₁-C₆-Alkylsulfonyl, Cyano, Nitro, C₁-C₆-Halogenalkyl, C₁-C₁₀-Alkyl, Hydroxyl, C₂-C₆-Alkenyl, C₁-C₆-Hydroxyalkyl, Carboxyl, C₃-C₇-Cycloalkyl, N-C₁-C₆-Alkylamino, Di-N-C₁-C₆-Alkylamino, C₁-C₆-Alkoxycarbonyl, Aminocarbonyl, C₁-C₆-Alkoxy, C₁-C₆-Halogenalkoxy, Sulfamyl, fünf- oder sechsgliedrigen Heterocyclen und Amino; oder eines pharmazeutisch zulässigen Salzes davon.

3. Verwendung nach Anspruch 2, bei welcher die Verbindung ausgewählt wird aus Verbindungen und ihren pharmazeutisch zulässigen Salzen der Gruppe, bestehend aus:
4-[5-(4-Chlorphenyl)-3-(trifluormethyl)-1H-pyrazol-1-yl]benzolsulfonamid;
4-[5-Phenyl-3-(trifluormethyl)-1H-pyrazol-1-yl]benzolsulfonamid;
4-[5-(4-Fluorphenyl)-3-(trifluormethyl)-1H-pyrazol-1-yl]benzolsulfonamid;
4-[5-(4-Methoxyphenyl)-3-(trifluormethyl)-1H-pyrazol-1-yl]-benzolsulfonamid;
4-[5-(4-Chlorphenyl)-3-(difluormethyl)-1H-pyrazol-1-yl]benzolsulfonamid;
4-[5-(4-Methylphenyl)-3-(trifluormethyl)-1H-pyrazol-1-yl]benzolsulfonamid;
4-[4-Chlor-5-(4-chlorphenyl)-3-(trifluormethyl)-1H-pyrazol-1 -yl]benzolsulfonamid;
4-[3-(Difluormethyl)-5-(4-methylphenyl)-1H-pyrazol-1-yl]benzolsulfonamid;
4-[3-(Difluormethyl)-5-phenyl-1H-pyrazol-1-yl]benzolsulfonamid;
4-[3-(Difluormethyl)-5-(4-methoxyphenyl)-1H-pyrazol-1-yl]benzolsulfonamid;
4-[3-Cyano-5-(4-fluorphenyl)-1H-pyrazol-1-yl]benzolsulfonamid;
4-[3-(Difluormethyl)-5-(3-fluor-4-methoxyphenyl)-1H-pyrazol-1-yl]benzolsulfonamid;
4-[5-(3-Fluor-4-methoxyphenyl)-3-(trifluormethyl)-1H-pyrazol-1-yl]benzolsulfonamid;
4-[4-Chlor-5-phenyl)-1H-pyrazol-1-yl]benzolsulfonamid;
4-[5-(4-Chlorphenyl)-3-(hydroxymethyl)-1H-pyrazol-1-yl]benzolsulfonamid; und
4-[5-(4-(N,N-Dimethylamino)phenyl)-3-(trifluormethyl)-1H-pyrazol-1-yl]benzolsulfonamid.

4. Verwendung nach Anspruch 2, bei welcher die Verbindung 4-[5-(4-Methylphenyl)-3-(trifluormethyl)-1H-pyrazol-1-yl]benzolsulfonamid oder ein pharmazeutisch zulässiges Salz davon ist.

5. Verwendung nach Anspruch 2, bei welcher die Verbindung 4-[5-(4-Chlorphenyl)-3-(difluormethyl)-1 H-pyrazol-1-yl]benzolsulfonamid oder eines pharmazeutisch zulässiges Salz davon ist.

6. Verwendung nach Anspruch 2, bei welcher die Verbindung 4-[5-(3-Fluor-4-methoxyphenyl)-3-(difluormethyl)-1H-pyrazol-1-yl]benzolsulfonamid oder ein pharmazeutisch zulässiges Salz davon ist.

7. Verwendung nach Anspruch 1, bei welcher die Demenz ausgewählt ist aus Alzheimer-Krankheit, vaskuläre Demenz, Multiinfarktdemenz, präsenile Demenz, Alkoholdemenz und senile Demenz.

8. Verwendung nach Anspruch 2, bei welcher die Demenz Alzheimer-Krankheit ist.

9. Verwendung einer Verbindung der Formel I 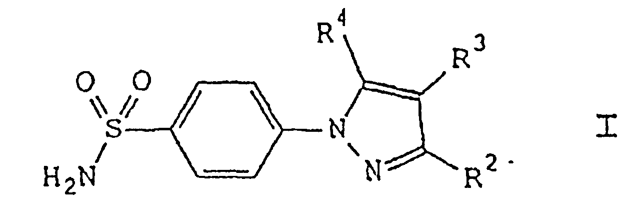 worin R² ausgewählt wird aus: Hydrido, Alkyl, Halogenalkyl, Alkoxycarbonyl, Cyano, Cyanoalkyl, Carboxyl, Aminocarbonyl, Alkylaminocarbonyl, Cycloalkylaminocarbonyl, Arylaminocarbonyl, Carboxyalkylaminocarbonyl, Carboxyalkyl, Aralkoxycarbonylalkylaminocarbonyl, Aminocarbonylalkyl, Alkoxycarbonylcyanoalkenyl und Hydroxyalkyl;
worin R³ ausgewählt wird aus: Hydrido, Alkyl, Cyano, Hydroxyalkyl, Cycloalkyl, Alkylsulfonyl und Halogen; sowie
worin R⁴ ausgewählt wird aus: Aralkenyl, Aryl, Cycloalkyl, Cycloalkenyl und Heterocyclen; worin R⁴ wahlweise in einer substituierbaren Stelle substituiert ist mit einem oder mehreren Resten, ausgewählt aus Halogen, Alkylthio, Alkylsulfonyl, Cyano, Nitro, Halogenalkyl, Alkyl, Hydroxyl, Alkenyl, Hydroxyalkyl, Carboxyl, Cycloalkyl, Alkylamino, Dialkylamino, Alkoxycarbonyl, Aminocarbonyl, Alkoxy, Halogenalkoxy, Sulfamyl, Heterocyclen und Amino;
oder ein pharmazeutisch zulässiges Salz davon zum Herstellen eines Medikamentes für die Prävention einer Demenz, ausgewählt aus Alzheimer-Krankheit, vaskuläre Demenz, Multiinfarktdemenz, präsenile Demenz, Alkoholdemenz und senile Demenz.

10. Verwendung nach Anspruch 9, bei welcher R² ausgewählt wird aus Hydrido, C₁-C₁₀-Alkyl, C₁-C₆-Halogenalkyl, C₁-C₆-Alkoxycarbonyl, Cyano, C₁-C₆-Cyanoalkyl, Carboxyl, Aminocarbonyl, C₁-C₆-N-Alkylaminocarbonyl, C₃-C₇-Cycloalkylaminocarbonyl, Arylaminocarbonyl, Carboxy-C₁-C₆-Alkylaminocarbonyl, Aminocarbonyl-C₁-C₆-Alkyl, Aryl-C₁-C₆-Alkoxycarbonylalkylaminocarbonyl, Carboxy-C₁-C₆-Alkyl, C₁-C₆-Alkoxycarbonylcyanoalkenyl und C₁-C₆-Hydroxyalkyl; worin R³ ausgewählt wird aus Hydrido, C₁-C₁₀-Alkyl, Cyano, C₁-C₆-Hydroxyalkyl, C₃-C₇-Cycloalkyl, C₁-C₆-Alkylsulfonyl und Halogen; und worin R⁴ ausgewählt wird aus Aralkenyl, Aryl, Cycloalkyl, Cycloalkenyl und Heterocyclen; worin R⁴ wahlweise an einer substituierbaren Stelle substituiert ist mit einem oder mehreren Resten, ausgewählt aus Halogen, C₁-C₆-Alkylthio, C₁-C₆-Alkylsulfonyl, Cyano, Nitro, C₁-C₆-Halogenalkyl, C₁-C₁₀-Alkyl, Hydroxyl, C₂-C₆-Alkenyl, C₁-C₆-Hydroxyalkyl, Carboxyl, C₃-C₇-Cycloalkyl, C₁-C₆-N-Alkylamino, C₁-C₆-N-Dialkylamino, C₁-C₆-Alkoxycarbonyl, Aminocarbonyl, C₁-C₆-Alkoxy, C₁-C₆-Halogenalkoxy, Sulfamyl, fünf- oder sechsgliedrigen Heterocyclen und Amino; oder eines pharmazeutisch zulässigen Salzes davon.

11. Verwendung nach Anspruch 10, bei welcher die Verbindung ausgewählt wird aus Verbindungen und ihren pharmazeutisch zulässigen Salzen der Gruppe, bestehend aus:
4-[5-(4-Chlorphenyl)-3-(trifluormethyl)-1H-pyrazol-1-yl]benzolsulfonamid;
4-[5-Phenyl-3-(trifluormethyl)-1H-pyrazol-1-yl]benzolsulfonamid;
4-[5-(4-Fluorphenyl)-3-(trifluormethyl)-1H-pyrazol-1-yl]benzolsulfonamid;
4-[5-(4-Methoxyphenyl)-3-(trifluormethyl)-1H-pyrazol-1-yl]-benzolsulfonamid;
4-[5-(4-Chlorphenyl)-3-(difluormethyl)-1H-pyrazol-1-yl]benzolsulfonamid;
4-[5-(4-Methylphenyl)-3-(trifluormethyl)-1H-pyrazol-1-yl]benzolsulfonamid;
4-[4-Chlor-5-(4-chlorphenyl)-3-(trifluormethyl)-1H-pyrazol-1-yl]benzolsulfonamid;
4-[3-(Difluormethyl)-5-(4-methylphenyl)-1 H-pyrazol-1-yl]benzolsulfonamid;
4-[3-(Difluormethyl)-5-phenyl-1H-pyrazol-1-yl]benzolsulfonamid;
4-[3-(Difluormethyl)-5-(4-methoxyphenyl)-1H-pyrazol-1-yl]benzolsulfonamid;
4-[3-Cyano-5-(4-fluorphenyl)-1H-pyrazol-1-yl]benzolsulfonamid;
4-[3-(Difluormethyl)-5-(3-fluor-4-methoxyphenyl)-1H-pyrazol-1-yl]benzolsulfonamid;
4-[5-(3-Fluor-4-methoxyphenyl)-3-(trifluormethyl)-1H-pyrazol-1-yl]benzolsulfonamid;
4-[4-Chlor-5-phenyl)-1H-pyrazol-1-yl]benzolsulfonamid;
4-[5-(4-Chlorphenyl)-3-(hydroxymethyl)-1H-pyrazol-1-yl]benzolsulfonamid; und
4-[5-(4-(N,N-Dimethylamino)phenyl)-3-(trifluormethyl)-1H-pyrazol-1-yl]benzolsulfonamid.

12. Verwendung nach Anspruch 10, bei welcher die Verbindung 4-[5-(4-Methylphenyl)-3-(trifluormethyl)-1H-pyrazol-1-yl]benzolsulfonamid oder ein pharmazeutisch zulässigen Salzes davon ist.

13. Verwendung nach Anspruch 10, bei welcher die Verbindung 4-[5-(4-Chlorphenyl)-3-(difluormethyl)-1H-pyrazol-1-yl]benzolsulfonamid oder ein pharmazeutisch zulässiges Salz davon ist.

14. Verwendung nach Anspruch 10, bei welcher die Verbindung 4-[5-(3-Fluor-4-methoxyphenyl)-3-(difluormethyl)-1H-pyrazol-1-yl]benzolsulfonamid oder ein pharmazeutisch zulässiges Salz davon ist.

## Revendications

1. Utilisation d'un composé de formule I : 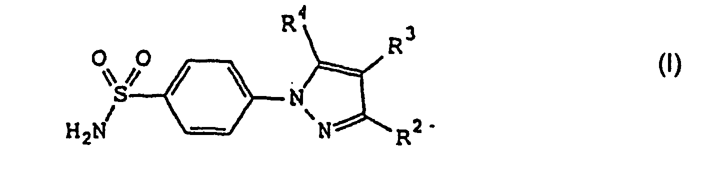 dans laquelle R² est choisi parmi les groupes hydrido, alkyle, haloalkyle, alcoxycarbonyle, cyano, cyanoalkyle, carboxyle, aminocarbonyle, alkylaminocarbonyle, cycloalkylaminocarbonyle, arylaminocarbonyle, carboxyalkylaminocarbonyle, carboxyalkyle, aralcoxycarbonylalkylaminocarbonyle, alcoxycarbonylcyanoalcényle et hydroxyalkyle;
dans laquelle R³ est choisi parmi les groupes hydrido, alkyle, cyano, hydroxyalkyle, cycloalkyle, alkylsulfonyle et halo; et
dans laquelle R⁴ est choisi parmi les groupes aralcényle, aryle, cycloalkyle, cycloalcényle et hétérocycliques; dans laquelle R⁴ est éventuellement substitué en une position substituable avec un ou plusieurs radicaux choisis parmi les groupes halo, alkylthio, alkylsulfonyle, cyano, nitro, haloalkyle, alkyle, hydroxyle, alcényle, hydroxyalkyle, carboxyle, cycloalkyle, alkylamino, dialkylamino, alcoxycarbonyle, aminocarbonyle, alcoxy, haloalcoxy, sulfamyle, hétérocycliques et amino;
ou d'un sel pharmaceutiquement acceptable de celui-ci, pour la préparation d'un médicament pour traiter une démence chez un sujet.

2. Utilisation suivant la revendication 1, dans laquelle R² est choisi parmi les groupes hydrido, alkyle en C₁-C₁₀, haloalkyle en C₁-C₆, alcoxycarbonyle en C₁-C₆, cyano, cyanoalkyle en C₁-C₆, carboxyle, aminocarbonyle, N-alkyl(C₁-C₆)aminocarbonyle, cycloalkyl(C₃-C₇)aminocarbonyle, arylaminocarbonyle, carboxyalkyl(C₁-C₆)aminocarbonyle, arylalcoxy(C₁-C₆)carbonylalkylaminocarbonyle, carboxyalkyle en C₁-C₆, alcoxy(C₁-C₆)carbonylcyanoalcényle et hydroxyalkyle en C₁-C₆; dans laquelle R³ est choisi parmi les groupes hydrido, alkyle en C₁-C₁₀, cyano, hydroxyalkyle en C₁-C₆, cycloalkyle en C₃-C₇, alkylsulfonyle en C₁-C₆ et halo; et dans laquelle R⁴ est choisi parmi les groupes aralcényle, aryle, cycloalkyle, cycloalcényle et hétérocycliques; dans laquelle R⁴ est éventuellement substitué en une position substituable avec un ou plusieurs radicaux choisis parmi les groupes halo, alkylthio en C₁-C₆, alkylsulfonyle en C₁-C₆, cyano, nitro, haloalkyle en C₁-C₆, alkyle en C₁-C₁₀, hydroxyle, alcényle en C₂-C₆, hydroxyalkyle en C₁-C₆, carboxyle, cycloalkyle en C₃-C₇, N-alkylamino en C₁-C₆, di-N-alkylamino en C₁-C₆, alcoxycarbonyle en C₁-C₆, aminocarbonyle, alcoxy en C₁-C₆, haloalcoxy en C₁-C₆, sulfamyle, hétérocycliques pentagonaux ou hexagonaux et amino; ou un sel pharmaceutiquement acceptable de celui-ci.

3. Utilisation suivant la revendication 2, dans laquelle le composé est choisi parmi les composés, et leurs sels pharmaceutiquement acceptables, du groupe comprenant :
le 4-[5-(4-chlorophényl)-3-(trifluorométhyl)-1H-pyrazol-1-yl]benzènesulfonamide,
le 4-[5-phényl-3-(trifluorométhyl)-1H-pyrazol-1-yl]benzènesulfonamide,
le 4-[5-(4-fluorophényl)-3-(trifluorométhyl)-1H-pyrazol-1-yl]benzènesulfonamide,
le 4-[5-(4-méthoxyphényl)-3-(trifluorométhyl)-1H-pyrazol-1-yl]benzènesulfonamide,
le 4-[5-(4-chlorophényl)-3-(difluorométhyl)-1H-pyrazol-1-yl]benzènesulfonamide,
le 4-[5-(4-méthylphényl)-3-(trifluorométhyl)-1H-pyrazol-1-yl]benzènesulfonamide,
le 4-[4-chloro-5-(4-chlorophényl)-3-(trifluorométhyl)-1H-pyrazol-1-yl]benzènesulfonamide,
le 4-[3-(difluorométhyl)-5-(4-méthylphényl)-1H-pyrazol-1 -yl]benzènesulfonamide,
le 4-[3-(difluorométhyl)-5-phényl-1H-pyrazol-1-yl]benzènesulfonamide,
le 4-[3-(difluorométhyl)-5-(4-méthoxyphényl)-1H-pyrazol-1-yl]benzènesulfonamide,
le 4-[3-cyano-5-(4-fluorophényl)-1H-pyrazol-1-yl]benzènesulfonamide,
le 4-[3-(difluorométhyl)-5-(3-fluoro-4-méthoxyphényl)-1H-pyrazol-1-yl]benzènesulfonamide,
le 4-[5-(3-fluoro-4-méthoxyphényl)-3-(trifluorométhyl)-1H-pyrazol-1-yl]benzènesulfonamide,
le 4-[4-chloro-5-phényl-1H-pyrazol-1-yl]benzènesulfonamide,
le 4-[5-(4-chlorophényl)-3-(hydroxyméthyl)-1H-pyrazol-1-yl]benzènesulfonamide, et
le 4-[5-(4-N,N-diméthylamino)phényl)-3-(trifluorométhyl)-1H-pyrazol-1-yl]benzènesulfonamide.

4. Utilisation suivant la revendication 2, dans laquelle le composé est le 4-[5-(4-méthylphényl)-3-(trifluorométhyl)-1H-pyrazol-1-yl]benzènesulfonamide, ou un sel pharmaceutiquement acceptable de celui-ci.

5. Utilisation suivant la revendication 2, dans laquelle le composé est le 4-[5-(4-chlorophényl)-3-(difluorométhyl)-1H-pyrazol-1 -yl]-benzènesulfonamide, ou un sel pharmaceutiquement acceptable de celui-ci.

6. Utilisation suivant la revendication 2, dans laquelle le composé est le 4-[5-(3-fluoro-4-méthoxyphényl)-3-(difluorométhyl)-1H-pyrazol-1-yl]benzènesulfonamide, ou un sel pharmaceutiquement acceptable de celui-ci.

7. Utilisation suivant la revendication 1, dans laquelle la démence est choisie parmi la maladie d'Alzheimer, le démence vasculaire, la démence multi-infarctus, la démence présénile, la démence alcoolique et la démence sénile.

8. Utilisation suivant la revendication 7, dans laquelle la démence est la maladie d'Alzheimer.

9. Utilisation d'un composé de formule I : 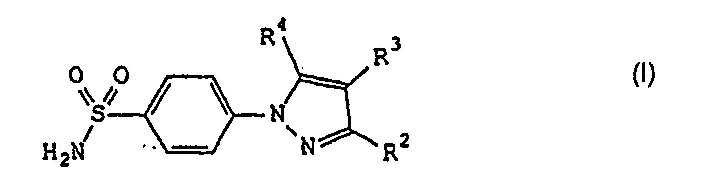 dans laquelle R² est choisi parmi les groupes hydrido, alkyle, haloalkyle, alcoxycarbonyle, cyano, cyanoalkyle, carboxyle, aminocarbonyle, alkylaminocarbonyle, cycloalkylaminocarbonyle, arylaminocarbonyle, carboxyalkylaminocarbonyle, carboxyalkyle, aralcoxycarbonylalkylaminocarbonyle, aminocarbonylalkyle, alcoxycarbonylcyanoalcényle et hydroxyalkyle;
dans laquelle R³ est choisi parmi les groupes hydrido, alkyle, cyano, hydroxyalkyle, cycloalkyle, alkylsulfonyle et halo; et
dans laquelle R⁴ est choisi parmi les groupes aralcényle, aryle, cycloalkyle, cycloalcényle et hétérocycliques; dans laquelle R⁴ est éventuellement substitué en une position substituable avec un ou plusieurs radicaux choisis parmi les groupes halo, alkylthio, alkylsulfonyle, cyano, nitro, haloalkyle, alkyle, hydroxyle, alcényle, hydroxyalkyle, carboxyle, cycloalkyle, alkylamino, dialkylamino, alcoxycarbonyle, aminocarbonyle, alcoxy, haloalcoxy, sulfamyle, hétérocycliques et amino;
ou d'un sel pharmaceutiquement acceptable de celui-ci, pour la préparation d'un médicament pour empêcher une démence choisie parmi la maladie d'Alzheimer, la démence vasculaire, la démence multi-infarctus, la démence présénile, la démence alcoolique et la démence sénile.

10. Utilisation suivant la revendication 9, dans laquelle R² est choisi parmi les groupes hydrido, alkyle en C₁-C₁₀, haloalkyle en C₁-C₆, alcoxycarbonyle en C₁-C₆, cyano, cyanoalkyle en C₁-C₆, carboxyle, aminocarbonyle, N-alkyl(C₁-C₆)aminocarbonyle, cycloalkyl(C₃-C₇)aminocarbonyle, arylaminocarbonyle, carboxyalkyl(C₁-C₆)aminocarbonyle, aminocarbonylalkyle en C₁-C₆, arylalcoxy(C₁-C₆)carbonylalkylaminocarbonyle, carboxyalkyle en C₁-C₆, alcoxy(C₁-C₆)carbonylcyanoalcényle et hydroxyalkyle en C₁-C₆; dans laquelle R³ est choisi parmi les groupes hydrido, alkyle en C₁-C₁₀, cyano, hydroxyalkyle en C₁-C₆, cycloalkyle en C₃-C₇, alkylsulfonyle en C₁-C₆ et halo; et dans laquelle R⁴ est choisi parmi les groupes aralcényle, aryle, cycloalkyle, cycloalcényle et hétérocycliques; dans laquelle R⁴ est éventuellement substitué en une position substituable avec un ou plusieurs radicaux choisis parmi les groupes halo, alkylthio en C₁-C₆, alkylsulfonyle en C₁-C₆, cyano, nitro, haloalkyle en C₁-C₆, alkyle en C₁-C₁₀, hydroxyle, alcényle en C₂-C₆, hydroxyalkyle en C₁-C₆, carboxyle, cycloalkyle en C₃-C₇, N-alkylamino en C₁-C₆, di-N-alkylamino en C₁-C₆, alcoxycarbonyle en C₁-C₆, aminocarbonyle, alcoxy en C₁-C₆, haloalcoxy en C₁-C₆, sulfamyle, hétérocycliques pentagonaux ou hexagonaux et amino; ou d'un sel pharmaceutiquement acceptable de celui-ci.

11. Utilisation suivant la revendication 10, dans laquelle le composé est choisi parmi les composés, et leurs sels pharmaceutiquement acceptables, du groupe comprenant :
le 4-[5-(4-chlorophényl)-3-(trifluorométhyl)-1H-pyrazol-1-yl]benzènesulfonamide,
le 4-[5-phényl-3-(trifluorométhyl)-1H-pyrazol-1-yl]benzènesulfonamide,
le 4-[5-(4-fluorophényl)-3-(trifluorométhyl)-1H-pyrazol-1-yl]benzènesulfonamide,
le 4-[5-(4-méthoxyphényl)-3-(trifluorométhyl)-1H-pyrazol-1-yl]benzènesulfonamide,
le 4-[5-(4-chlorophényl)-3-(difluorométhyl)-1H-pyrazol-1-yl]benzènesulfonamide,
le 4-[5-(4-méthylphényl)-3-(trifluorométhyl)-1H-pyrazol-1-yl]benzènesulfonamide,
le 4-[4-chloro-5-(4-chlorophényl)-3-(trifluorométhyl)-1H-pyrazol-1-yl]benzènesulfonamide,
le 4-[3-(difluorométhyl)-5-(4-méthylphényl)-1H-pyrazol-1-yl]benzènesulfonamide,
le 4-[3-(difluorométhyl)-5-phényl-1 H-pyrazol-1-yl]benzènesulfonamide,
le 4-[3-(difluorométhyl)-5-(4-méthoxyphényl)-1H-pyrazol-1-yl]benzènesulfonamide,
le 4-[3-cyano-5-(4-fluorophényl)-1H-pyrazol-1-yl]benzènesulfonamide,
le 4-[3-(difluorométhyl)-5-(3-fluoro-4-méthoxyphényl)-1H-pyrazol-1-yl]benzènesulfonamide,
le 4-[5-(3-fluoro-4-méthoxyphényl)-3-(trifluorométhyl)-1H-pyrazol-1-yl]benzènesulfonamide,
le 4-[4-chloro-5-phényl-1H-pyrazol-1-yl]benzènesulfonamide,
le 4-[5-(4-chlorophényl)-3-(hydroxyméthyl)-1H-pyrazol-1-yl]benzènesulfonamide, et
le 4-[5-(4-N,N-diméthylamino)phényl)-3-(trifluorométhyl)-1H-pyrazol-1-yl]benzènesulfonamide.

12. Utilisation suivant la revendication 10, dans laquelle le composé est le 4-[5-(4-méthylphényl)-3-(trifluorométhyl)-1H-pyrazol-1-yl]-benzènesulfonamide, ou un sel pharmaceutiquement acceptable de celui-ci.

13. Utilisation suivant la revendication 10, dans laquelle le composé est le 4-[5-(4-chlorophényl)-3-(difluorométhyl)-1H-pyrazol-1-yl]-benzènesulfonamide, ou un sel pharmaceutiquement acceptable de celui-ci.

14. Utilisation suivant la revendication 10, dans laquelle le composé est le 4-[5-(3-fluoro-4-méthoxyphényl)-3-(difluorométhyl)-1H-pyrazol-1-yl]benzènesulfonamide, ou un sel pharmaceutiquement acceptable de celui-ci.
